# EUROPÄISCHE PATENTANMELDUNG

(11) **EP 2 399 510 A2**
(43) Veröffentlichungstag der Anmeldung: **28.12.2011**
(21) Anmeldenummer: 11163817.7
(22) Anmeldetag: 27.04.2011
(51) Int. Cl.: A61B 5/00, H02J 17/00

(54) **Induktive Drehübertrager für Computertomographen mit Ripplekompensation**

(30) Priorität: 27.04.2010 DE 102010016652
(71) Anmelder: Schleifring und Apparatebau GmbH, 82256 Fürstenfeldbruck (DE)
(72) Erfinder: Zimpfer, Arno, 82291, Mammendorf (DE); Fischer, Stefan, 82256, Fürstenfeldbruck (DE)
(74) Vertreter: Lohr, Georg

(57) **Zusammenfassung**

Die Erfindung betrifft ein Verfahren zur Kompensation von Eingangsspannungsschwankungen in einem induktiven Koppler. Dieser weist einen Leistungsgenerator auf, der eine Wechselspannung in einen Serienresonanzkreis aus einem Resonanzkondensators und einem induktiven Drehübertrager einspeist. Der Leistungsgenerator wird von einer Eingangsgleichspannung gespeist, die beispielsweise aufgrund einer Restwelligkeit schwanken kann. Eine Steuereinheit ermittelt diese Eingangsgleichspannung und berechnet daraus eine optimale Arbeitsfrequenz für den Leistungsgenerator, so dass die Ausgangspannung an der Last konstant bleibt.

## Beschreibung

### Technisches Gebiet

Die Erfindung bezieht sich auf induktive Koppler zur kontaktlosen Leistungsübertragung, insbesondere induktive Drehübertrager und hier besonders induktive Drehübertrager für Computertomographen. Durch diese wird die überwiegend zum Betrieb der Röntgenröhre notwendige elektrische Leistung von der stationären Seite zur rotierenden Seite der Gantry eines Computertomographen übertragen. Die Übertragung erfolgt hier berührungslos mittels eines induktiven Drehübertragers, welcher ähnlich wie ein Transformator aufgebaut ist, bei dem die Primärseite und Sekundärseite gegeneinander drehbar sind.

### Stand der Technik

Bei gegeneinander beweglichen Einheiten, wie Radaranlagen oder auch Computertomographen, ebenso wie bei linear beweglichen Einheiten wie Krananlagen oder Förderfahrzeugen ist es häufig notwendig, elektrische Energie zwischen beweglichen Einheiten zu übertragen. Um diese Energie berührungslos zu übertragen, werden bevorzugt induktive Koppler eingesetzt. Diese haben gegenüber mechanischen Schleifbahnen oder auch Schleifringen den Vorteil dass Abrieb, Verschleiß, mechanischer Kraftaufwand zur Bewegung des Kopplers und auch der Wartungsaufwand wesentlich geringer sind. Der Begriff eines induktiven Kopplers bezieht sich hier auf eine Schaltung zur Erzeugung einer Wechselspannung zusammen mit einem induktiven Übertrager beziehungsweise Drehübertrager zur Energieübertragung zwischen zwei gegeneinander beweglichen und insbesondere drehbaren Teilen.

Induktive Drehübertrager, wie sie beispielsweise in der US 7,197,113 offenbart sind, haben magnetische Kerne aus Eisen- oder Ferritmaterial und wenigstens eine Wicklung auf jeder Seite der gegeneinander drehbaren Einheiten. In eine erste Wicklung wird ein Wechselstrom eingespeist und über eine zweite, gegenüber dieser beweglichen Wicklung wieder abgegriffen.

Die US 7,054,411 zeigt eine vollständige Schaltung eines induktiven Leistungsübertragungssystems für Computertomographen mit der zugehörigen Leistungselektronik.

In der DE 10 2007 006 394 A1 ist ein induktiver Drehübertrager offenbart, bei dem die Ausgangsspannung durch eine Steuereinheit geregelt wird, die hierzu eine elektrische Kenngröße an der Primärwicklung des Drehübertragers ermittelt.

Bei galvanisch gekoppelten Schleifringen ist es einfach, eine vordefinierte Spannung von der Statorseite auf die Rotorseite zu übertragen. Es müssen hier nur die relativ geringen ohmschen Verluste berücksichtigt werden. Bei induktiven Drehübertragern spielt die Streuinduktivität des Drehübertragers eine wesentliche Rolle. Sie stellt eine Frequenzabhängige Impedanz dar, die die Übertragungseigenschaften des Drehübertragers wesentlich beeinflusst. Diese Streuinduktivität hängt von verschiedenen Faktoren, wie der Induktivität der Wicklungen der Statorseite und der Rotorseite sowie vom magnetischen Aufbau ab. Um nun elektrische Energie über einen solchen Drehübertrager zu übertragen, wird zur Kompensation eine Serienkapazität in Reihe geschaltet. Hierdurch ergibt sich ein Serienresonanzkreis. Dieser hat bei seiner Resonanzfrequenz eine Impedanz von Null und ermöglicht hier die Übertragung großer Leistungen. Zur Steuerung des Leistungsflusses kann die Arbeitsfrequenz von der Resonanzfrequenz abweichend gewählt werden.

Statt eines Serienresonanzkreises kann durch Parallelschalten einer Kapazität auch ein Parallelresonanzkreis aufgebaut werden. Die im Folgenden beschriebenen Eigenschaften treffen ebenso auf einen Parallelresonanzkreis zu. Der Resonanzkreis hat bei seiner Resonanzfrequenz eine Impedanz von beinahe Null und ermöglicht hier die Übertragung großer Leistungen. Durch eine Änderung der Impedanz, welche durch eine Änderung der Schaltfrequenz herbeigeführt wird, kann die Ausgangsspannung gesteuert werden.

Wird die Anordnung nun bei einer für bestimmte Lastverhältnisse optimalen Schaltfrequenz betrieben, so ergibt sich eine Umsetzung der Ausgangspannung an der Last in einem bestimmten Verhältnis zur Eingangsspannung vom Netz. In den meisten Fällen ist die Eingangsspannung vom Netz eine gut gesiebte Gleichspannung, die meist sogar von einer Leistungsfaktorkorrekturschaltung stammt. In manchen Anwendungen wird jedoch eine solche Leistungsfaktorkorrekturschaltung nicht benötigt und kann entfallen. Damit steht als Eingangsspannung für den Drehübertrager nur eine gleichgerichtete Wechselspannung mit relativ hoher Restwelligkeit zur Verfügung, die wieder in einer entsprechenden Restwelligkeit der Ausgangspannung an der Last resultiert. Um die Welligkeit der Spannung an der Last zu reduzieren kann beispielsweise die Ausgangsspannung gemessen und dieser Messwert auf die stationäre Seite zurückgeführt werden. Bei konventionellen (nicht beweglichen) Schaltnetzteilen wird hierfür ein separater Rückkoppeltransformator eingesetzt. Bei Drehübertragern wird aber ein zusätzlicher Drehübertrager benötigt, der zusätzliche Kosten verursacht und Platz benötigt.

Alternativ können die eingangsseitigen Siebkondensatoren vergrößert werden. Dies führt aber zu einem größeren Volumen der Anordnung und höheren Kosten. Zudem steigt die Primärseitige Spitzenstromaufnahme aus dem Netz, wodurch die Oberwellen im Netz erhöht werden.

Eine andere Möglichkeit ist, auf der rotierenden Seite eine zusätzliche Wandlerstufe zwischen Sekundärseite des rotierenden Transformators und Ausgang einzubauen. Für diese Wandlerstufen werden häufig Tief- oder Hochsetzsteller verwendet, aber auch andere Wandler, wie Zeta- oder Cuk- Wandler sind möglich. Die Eingangsspannung dieser nachgeschalteten Wandlerstufe kann in einem großen Bereich schwanken, wobei die Ausgangsspannung konstant gehalten wird. Diese Lösung benötigt aber auf der rotierenden Seite einen zusätzlichen Wandler, der die Kosten und auch Gewicht sowie Volumen der Anordnung erhöht.

### Darstellung der Erfindung

Der Erfindung liegt die Aufgabe zu Grunde, eine induktive Koppeleinrichtung zur Übertragung elektrischer Energie zwischen zwei gegeneinander beweglichen Einheiten, insbesondere einem induktiven Drehübertrager derart auszugestalten, dass durch eine primärseitige Steuerung die Ausgangsspannung unabhängig von Schwankungen der Eingangsspannung konstant gehalten werden kann. So kann auch die Größe eines eingangsseitigen Siebkondensators verringert werden. Ein weiterer Aspekt der Erfindung ist ein Verfahren, um die Ausgangspannung eines induktiven Kopplers unabhängig von Schwankungen der Eingangsspannung konstant zu halten. Weiterhin soll entsprechend der Erfindung kein Drehübertrager zur Übertragung von Steuersignalen von der Sekundärseite zur Primärseite zur Übertragung von Messwerten der Ausgangspannung auf der Sekundärseite notwendig sein.

Diese Aufgabe wird durch eine Vorrichtung nach Anspruch 1 gelöst. Vorteilhafte Ausgestaltungen der Erfindung sind in den Unteransprüchen angegeben.

Eine erfindungsgemäße induktive Koppeleinrichtung, vorzugsweise ein induktiver Drehübertrager umfasst einen Leistungsgenerator zur Erzeugung einer gepulsten Gleichspannung oder einer Wechselspannung. Weiterhin umfasst sie einen induktiven Leistungsübertrager mit einer Primärseite und eine Sekundärseite, die gegeneinander beweglich sind. Es weist eine Primärseite wenigstens eine Primärwicklung und die Sekundärseite wenigstens eine Sekundärwicklung auf. Weiterhin ist in Serie oder parallel zur Primärwicklung wenigstens eine Kapazität, vorzugsweise in Form eines Resonanzkondensators geschaltet, so dass sich ein Serienresonanzkreis ergibt. Alternativ kann auch eine Kapazität parallel zur Primärwicklung geschaltet sein, um einen parallel Resonanzkreis zu erhalten. Die gepulste Gleichspannung bzw. Wechselspannung des Leistungsgenerators wird zur Leistungsübertragung in diesen Resonanzkreis eingespeist, so dass in der Primärwicklung ein wechselndes Magnetfeld entsteht. Dieses induziert in der Sekundärwicklung wiederum einen Strom, welcher an eine Last abgegeben wird. Hierbei kann optional noch ein Gleichrichter wahlweise mit Glättungs- und oder Filterelementen zwischen Sekundärwicklung und Last geschaltet sein. Zur besseren Verkopplung zwischen Primärwicklung und Sekundärwicklung sind Bauteile mit weichmagnetischen Materialien, vorzugsweise Eisen- oder Ferritbauteile vorgesehen. Optional kann zwischen dem Leistungsgenerator und dem induktiven Leistungsübertrager noch ein Anpasstransformator vorgesehen sein.

Der Leistungsgenerator wird von einer Gleichspannung gespeist. Diese Gleichspannung kann beispielsweise durch Gleichrichtung und Siebung mit einem Ladekondensator aus einer Wechselspannung, beispielsweise einer Netzspannung gewonnen werden. Zur Steuerung des Leistungsgenerators ist eine Steuereinheit vorgesehen. Diese Steuereinheit ermittelt den Wert der Gleichspannung zur Speisung des Leistungsgenerators und stellt die Frequenz des Leistungsgenerators entsprechend ein. Durch eine Änderung der Frequenz des Leistungsgenerators kann der Leistungstranfer über den Resonanzkreis beeinflusst werden, da der Scheinwiderstand des Resonanzkreises frequenzabhängig ist. Die Funktion, auch mathematische Funktion genannt (der Zusammenhang) zwischen der Frequenz des Leistungsgenerators und der Gleichspannung zur Speisung des Leistungsgenerators ist bevorzugt in einer Berechnungsformel und/oder einer Wertetabelle festgehalten. Hierin können optional noch weitere Parameter, wie beispielsweise der Laststrom mit berücksichtigt werden. Es wird nun die Frequenz des Leistungsgenerators in Abhängigkeit von der Gleichspannung so eingestellt, dass die Lastspannung an der Last möglichst konstant und damit unabhängig von der Gleichspannung ist. Bevorzugterweise erfolgte Einstellung derart, dass die Lastspannung auch unabhängig von anderen Parametern, wie beispielsweise des Laststroms oder auch der Lastspannung ist.

Bevorzugt wird die mathematische Funktion zwischen der Frequenz des Leistungsgenerators und der Gleichspannung zur Speisung des Leistungsgenerators an einem Mustergerät (Prototypen, Funktionsmuster, Nullseriengerät, etc.) ermittelt und in einem Speicher abgespeichert. Alternativ kann diese Funktion aber auch im Rahmen eines Kalibrierlaufs ermittelt werden.

Ein erfindungsgemäßes Verfahren dient zur Erzeugung einer konstanten Ausgangspannung einer induktiven Koppeleinrichtung zur Übertragung elektrischer Energie zwischen zwei gegeneinander beweglichen Einheiten mit einem Leistungsgenerator sowie einem induktiven Leistungsübertrager und wenigstens einer Resonanzkapazität wie oben beschrieben. Es umfasst die Schritte:
a) Messen der Eingangsgleichspannung des Leistungsgenerators;
b) Ermitteln einer Frequenz mittels wenigstens einer Berechnungsformel und/oder Wertetabelle in Abhängigkeit von der gemessenen Eingangsgleichspannung;
c) Einstellen der Frequenz des Leistungsgenerators auf die ermittelte Frequenz, so dass die Lastspannung an der Last weitgehend unabhängig von der Eingangsgleichspannung ist.

Die Schritte a) bis c) werden bevorzugt in dieser Sequenz kontinuierlich durchgeführt. Die Wiederholfrequenz dieser Sequenz soll so hoch sein, dass beispielsweise ein Eingangsspannungsripple präzise abgetastet und entsprechend ausgelegt werden kann. Bei einer Netzfrequenz von 50 Hz hat sich eine Wiederholfrequenz von 1000 Hz als vorteilhaft erwiesen. Selbst verständlich sind auch höhere oder Niedrigere Wiederholfrequenzen anwendbar.

Weiterhin kann das Verfahren bevorzugt ergänzt werden durch
d) Erzeugen einer Berechnungsformel und/oder Wertetabelle, die die Funktion (den Zusammenhang) zwischen Lastspannung und Eingangsgleichspannung wiedergibt;

Das Ergebnis wird bevorzugt in einem Speicher gespeichert. Der Schritt d) wird bevorzugt vor dem eigentlichen Betrieb (der Aktivierung) der induktiven Koppeleinrichtung, das heißt vor der Leistungsübertragung oder auch schon in einer Produktionsphase des Gerätes oder einer späteren Kalibrierphase durchgeführt.

Das Erfindungsgemäße Verfahren ist auch für induktive Drehübertrager mit mehreren Primärwicklungen und/oder mehreren Sekundärwicklungen anwendbar.

Ein weiterer Gegenstand der Erfindung ist ein Computertomograph mit einer zuvor beschriebenen induktiven Koppeleinrichtung.

Die in diesem Dokument gemachten Ausführungen beziehen sich der Anschaulichkeit halber auf induktive Drehübertrager zur Energieübertragung zwischen gegeneinander drehbaren Einheiten, insbesondere bei Computertomographen. Dies ist auch der wesentliche Gegenstand der Erfindung. Es ist für den Fachmann jedoch klar, dass dieselben Prinzipien auch für beliebige Drehübertrager, und/oder auch für eine berührungslose Energieübertragung zwischen beliebig gegeneinander beweglichen Einheiten, insbesondere zwischen linear beweglichen Einheiten einsetzbar sind. Es muss hier lediglich eine Anpassung der Geometrie des Drehübertragers an die Bahn und die Art der Bewegung vorgenommen werden. Das erfindungsgemäße Verfahren ist hier genauso vorteilhaft einsetzbar. Grundsätzlich ist die Erfindung für induktive Koppeleinrichtungen aller Leistungsklassen geeignet. Besonders günstig ist sie jedoch in Leistungsklassen von mehreren Kilowatt bis über 100 Kilowatt einsetzbar.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.
Figur 1 zeigt eine erfindungsgemäße Vorrichtung.
Figur 2 zeigt eine Prinzipdarstellung eines Computertomographen.
Figur 3 zeigt den Verlauf der Lastspannung nach dem Stand der Technik.
Figur 4 zeigt den Verlauf der Lastspannung entsprechend der Erfindung.

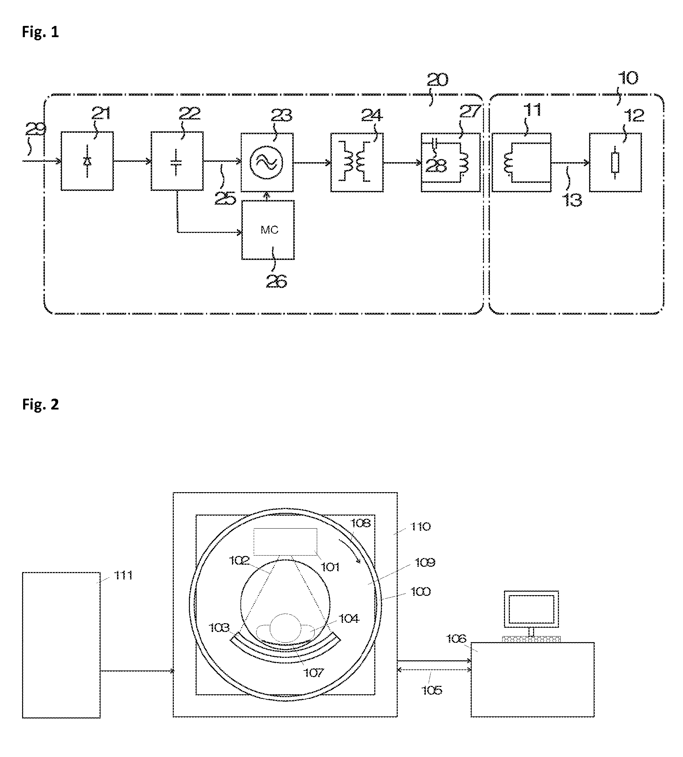

In Figur 1 ist eine induktive Koppeleinrichtung zur Übertragung elektrischer Energie zwischen zwei gegeneinander beweglichen Einheiten entsprechend der Erfindung dargestellt. Das stationäre Teil 20 ist über einen drehbaren Transformator mit einer Primärseite 27 und einer Sekundärseite 11 mit dem rotierenden Teil 10 verbunden. Der stationäre Teil erhält elektrische Energie über den Netzeingang 29. Dies könnte beispielsweise ein 3-Phasen AC Eingang mit 3*400V AC / 50 Hz sein. Die Eingangsspannung wird über den Netzgleichrichter 21 gleichgerichtet. Nach dem Netzgleichrichter ist einer oder mehrere Kondensatoren angeordnet, um die Welligkeit (ripple) der gleichgerichteten Eingangsspannung zu reduzieren. Die so geglättete Eingangsgleichspannung 25 wird dem Leistungsgenerator zugeführt. Der Leistungsgenerator 23 generiert eine Wechselspannung oder eine pulsierende Gleichspannung. Er weist bevorzugt eine Schaltstufe wie eine Halbbrücken- oder Vollbrückenschaltung auf. Zur Anpassung der Ausgangspannung beziehungsweise der Impedanzen kann ein optionaler Anpasstransformator 24 vorgesehen sein. Ein weiterer Vorteil eines solchen Anpasstransformators kann die zusätzliche Potenzialtrennung sein. In Serie mit der Primärseite des drehbaren Transformators 27 ist ein Serienkondensator 28 geschaltet, um einen Serienresonanzkreis zu bilden. Grundsätzlich hat diese Schaltung mehrere Resonanzen, wobei es aber nur eine Serienresonanzfrequenz gibt, bei der die gesamte Schaltung einen äußerst niedrigen Serienwiderstand zwischen Primärseite und Sekundärseite aufweist. Die Übertragung der elektrischen Energie von der Primärseite auf die Sekundärseite kann somit durch die Schaltfrequenz des Leistungsgenerators gesteuert werden. An dem rotierenden Teil 10 liefert die Sekundärseite des drehbaren Transformators 11 eine Lastspannung 13 an die Last 12.

Eine Steuereinheit 26, beispielsweise in Form eines Microcontrollers steuert den Leistungsgenerator 23. Die Steuereinheit ermittelt den Wert der Eingangsgleichspannung 25 und stellt die Frequenz des Leistungsgenerators derart ein, dass die Lastspannung 13 weit gehend unabhängig von der Eingangsgleichspannung ist. Hierzu hat die Steuereinheit eine Funktion und/oder Wertetabelle zur Darstellung der Abhängigkeit der Frequenz von der gemessenen Eingangsgleichspannung. Zusätzlich kann die Steuereinheit noch weitere Parameter, wie den Eingangsstrom zur Steuerung des Leistungsgenerators heranziehen.

In Figur 2 ist noch schematisch der Aufbau eines Computertomographen mit einem erfindungsgemäßen induktiven Drehübertrager dargestellt. Der stationäre Teil des Drehübertragers ist in einem massiven Rahmen 110 aufgehängt. Der rotierende Teil der Gantry 109 ist gegenüber diesem drehbar gelagert und dreht sich in Drehrichtung 108. Dort befindet sich eine Röntgenröhre 101 die ein Röntgenstrahlenbündel 102 erzeugt, welches den auf einer Liege 107 liegenden Patienten 104 durchstrahlt und von einem Detektor 103 aufgefangen und in elektrische Signale umgewandelt wird. Zur Übertragung der elektrischen Energie aus einer Energieversorgungseinheit 111 ist eine induktive Leistungsübertragungsstrecke 100, auch induktiver Koppler mit einem induktiven Drehübertrager vorgesehen. Die Primärseite hier an dem stationären Teil und die Sekundärseite an dem rotierenden Teil angeordnet. Die von dem Detektor 103 ermittelten Daten werden an eine Auswerteeinheit 106 übertragen. Hierzu dient ein Steuerbus 105 mit dem auch von der Auswerteeinheit die Gantry selbst gesteuert werden kann.

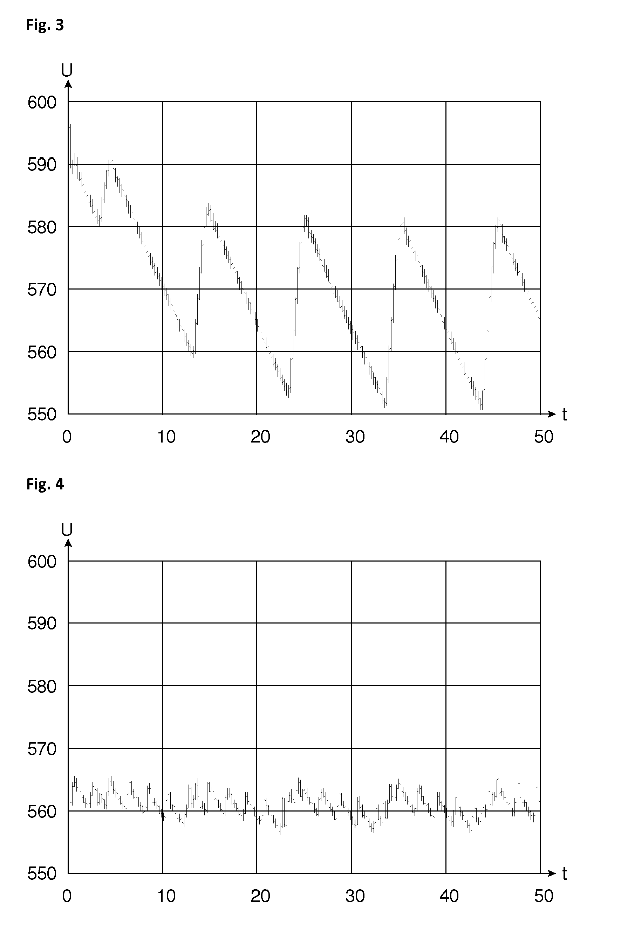

In Figur 3 ist der Verlauf der Lastspannung nach dem Stand der Technik dargestellt. Es ist hier die Lastspannung U in einem Bereich von 550 bis 600 Volt über eine Zeit t von 0 bis 50 Millisekunden aufgetragen. Es ist hier deutlich die Welligkeit (Ripple) in der Lastspannung mit einer Amplitude von circa 30 Volt zu erkennen.

In der Figur 4 ist der Verlauf der Lastspannung nach der Erfindung dargestellt. Die Skalierung ist wie im vorhergehenden Diagramm. Durch die Erfindung wird die Welligkeit der Lastspannung auf eine Amplitude von circa 5 Volt reduziert. Es handelt sich hierbei nur um eine beispielhafte Darstellung mit simulierten Werten. Je nach konkreter Ausführungsform können sich gegenüber dem der Technik größere oder kleinere Verbesserungen ergeben.

### Bezugszeichenliste

- 10: rotierendes Teil
- 11: Sekundärseite des drehbaren Transformators
- 12: Last
- 13: Lastspannung
- 20: stationäres Teil
- 21: Netzgleichrichter
- 22: Glättungskondensator
- 23: Leistungsgenerator
- 24: Transformator
- 25: Eingangsgleichspannung
- 26: Steuereinheit
- 27: Primärseite des drehbaren Transformators
- 28: Serienkondensator
- 29: Netzeingang
- 100: Induktiver Leistungsübertrager
- 101: Röntgenröhre
- 102: Röntgenstrahlenbündel
- 103: Detektor
- 104: Patient
- 105: Steuerbus
- 106: Auswerteeinheit
- 107: Liege
- 108: Drehrichtung
- 109: Rotierender Teil der Gantry
- 110: Stationärer Rahmen der Gantry

## Patentansprüche

1. Induktive Koppeleinrichtung zur Übertragung elektrischer Energie zwischen zwei gegeneinander beweglichen Einheiten umfassend
- einen Leistungsgenerator (23) zur Erzeugung eines gepulsten Gleichspannung oder einer Wechselspannung aus einer Eingangsgleichspannung,
- wenigstens einen Serienresonanzkreis und/oder Parallelresonanzkreis mit wenigstens einem Resonanzkondensator (24) und einem induktiven Leistungsübertrager (11, 21) zur Kopplung der elektrischen Energie zwischen den beweglichen Einheiten zur Speisung einer Last mit einer Lastspannung und/oder Laststrom,
- eine Steuereinheit zur Steuerung und Erzeugung der Frequenz des Leistungsgenerators ,
**dadurch gekennzeichnet, dass**
die Steuereinheit zur Ermittlung der Eingangsgleichspannung ausgebildet ist und weiterhin Mittel zur Einstellung des der Frequenz des Leistungsgenerators in Abhängigkeit von der Eingangsgleichspannung derart aufweist, dass die Lastspannung und/oder der Laststrom weitgehend konstant bleibt.

2. Induktive Koppeleinrichtung nach Anspruch 1,
**dadurch gekennzeichnet, dass**
der Steuereinheit zur Ermittlung der Frequenz des Leistungsgenerators aus der Lastspannung wenigstens eine mathematische Funktion und/oder Wertetabelle zur Verfügung steht.

3. Induktive Koppeleinrichtung nach einem der vorhergehenden Ansprüche,
**dadurch gekennzeichnet, dass**
die Steuereinheit zur Ermittlung der Frequenz des Leistungsgenerators weitere Parameter wie den Eingangsstrom, den Laststrom und/oder die Lastspannung heranzieht.

4. Induktive Koppeleinrichtung einem der vorhergehenden Ansprüche,
**dadurch gekennzeichnet, dass**
die mathematische Funktion und/oder Wertetabelle an einem Mustergerät oder an der induktiven Koppeleinrichtung selbst im Rahmen eines Kalibrierlaufs ermittelt wurde.

5. Verfahren zur Erzeugung einer konstanten Ausgangspannung einer Induktiven Koppeleinrichtung zur Übertragung elektrischer Energie zwischen zwei gegeneinander beweglichen Einheiten umfassend
- einen Leistungsgenerator (23) zur Erzeugung eines gepulsten Gleichspannung oder einer Wechselspannung aus einer Eingangsgleichspannung,
- wenigstens einen Serienresonanzkreis und/oder Parallelresonanzkreis mit wenigstens einem Resonanzkondensator (24) und einem induktiven Leistungsübertrager (11, 21) zur Kopplung der elektrischen Energie zwischen den beweglichen Einheiten zur Speisung einer Last mit einer Lastspannung und/oder Laststrom,
- eine Steuereinheit zur Steuerung und Erzeugung der Frequenz des Leistungsgenerators ,
umfassend die folgenden Schritte:
a) Messen der Eingangsgleichspannung;
b) Ermitteln einer Frequenz mittels wenigstens einer mathematische Funktion und/oder Wertetabelle in Abhängigkeit von der gemessenen Eingangsgleichspannung derart, dass die Lastspannung und/oder der Laststrom weitgehend konstant bleibt;
c) Einstellen der Frequenz des Leistungsgenerators auf die ermittelte Frequenz;

6. Verfahren nach Anspruch 5,
**dadurch gekennzeichnet, dass**
vor der Aktivierung der induktiven Koppeleinrichtung folgender Verfahrensschritt durchgeführt wird:
d) Erzeugen einer mathematische Funktion und/oder Wertetabelle, die den Zusammenhang zwischen Lastspannung und Eingangsgleichspannung wiedergibt;

7. Computertomograph mit einer Induktiven Koppeleinrichtung nach einem der Ansprüche 1 bis 4.
